# EUROPÄISCHE PATENTSCHRIFT

(11) **EP 2 219 663 B1**
(45) Veröffentlichungstag und Bekanntmachung des Hinweises auf die Patenterteilung: **28.11.2012**
(21) Anmeldenummer: 08858971.8
(22) Anmeldetag: 12.12.2008
(51) Int. Cl.: A61K 38/19, C07K 14/525

(54) **REVERSES PROTEIN**
REVERSE PROTEIN
PROTÉINE EN PHASE INVERSE

(30) Priorität: 12.12.2007 AT 20152007
(43) Veröffentlichungstag der Anmeldung: 25.08.2010
(73) Patentinhaber: Apeptico Forschung Und Entwicklung Gmbh, 1050 Wien (AT)
(72) Erfinder: FISCHER, Bernhard, A-1160 Wien (AT); LUCAS, Rudolf, B-2630 Aartselaar (BE)
(74) Vertreter: Schwarz & Partner
(86) Internationale Anmeldenummer: PCT/AT2008/000447
(87) Internationale Veröffentlichungsnummer: WO 2009/073908

(56) Entgegenhaltungen:
- WO-A1-00/09149
- DATABASE Geneseq [Online] 18. November 2004 (2004-11-18), "Target molecule binding peptide #91." XP002516878 gefunden im EBI accession no. GSN:ADR75507 Database accession no. ADR75507 -& DATABASE Geneseq [Online] 18. November 2004 (2004-11-18), "Target molecule binding peptide #49." XP002516879 gefunden im EBI accession no. GSN:ADR75465 Database accession no. ADR75465 -& EP 1 452 868 A2 (PEPSCAN SYSTEMS B V [NL]) 1. September 2004 (2004-09-01)

## Beschreibung

Die vorliegende Erfindung betrifft ein Reverses Protein, nämlich ein Tumor Nekrose Faktor-Protein (Polypeptid), das N-terminal aus einem oder mehreren C-terminalen Teilen der Aminosäuresequenz des reifen Tumor Nekrose Faktors (TNF), und C-terminal aus einem oder mehreren N-terminalen Teilen der Aminosäuresequenz des TNF zusammengesetzt ist, wobei das Protein als Medikament verwendet werden kann, z.B. zur Behandlung von Lungenödemen.

Der Flüssigkeitstransport durch Zellschichten und Gewebe beruht primär auf einem osmotischen Gradienten durch einen aktiven vektoriellen Ionentransport, z.B. Natriumtransport. Dieser wird hauptsächlich durch streng regulierten und vital bedeutenden Ionenkanäle, wie z.B. dem Epithelial Natrium- Kanal Komplex (ENaC), bewerkstelligt. Wasser folgt diesem Gradienten passiv, unter anderem durch spezielle Wasserkanäle, wie dem Wasserkanal Aquaporin V. Für das Lungengewebe ist bekannt, dass basolateral an den pumpenden Zellen Na+/K+ ATPasen den vektoriellen Transport von Natrium ins Interstitium betreiben und schließlich die Ionen in die Lymph- und Blutgefässe an. Dieser Transport ist also aktiv und erfolgt unabhängig vom transpulmonalen Druck und der alveolären Proteinkonzentration.

Ein Ödem ist eine pathologische Flüssigkeitsansammlung in einem Organ, wie z.B. der Lunge, aber auch dem Gehirn oder der Haut. Bei einem Ödem in der Lunge spricht man von einem Lungenödem. Das Lungenödem basiert meist auf dem Ungleichgewicht zwischen Flüssigkeitsextravasation und Flüssigkeitsrückresorption. Sehr oft ist auch die Permeabilität des Lungengewebes geschädigt, sodass eine vermehrte Flüssigkeitszufuhr stattfindet, und sich die Flüssigkeit in den Lungenblässchen (Alveolen) ansammelt..

Eine solche Permeabilitätsstörung als Folge eines fehlenden Rücktransportes von Flüssigkeit aus den Lungenbläschen in das Interstitium ist besonders bedeutsam bei der Akuten Lungen Verletzung (engl. Acute Lung Injury, ALI) oder beim Akuten Respiratorischem Distress Syndrom (engl. Acute Respiatory Distress Syndrome, ARDS) oder beim schweren akuten Atemnotsyndrom (SARS), bei der Pneumonie und beim Multiorganversagen. Die Permeabilitätsstörung spielt aber auch eine Rolle bei anderen Lungenerkrankungen, wie Beatmung-induzierter Lungenschäden, Lungentransplantationen, Transfusion-assoziierten Lungenschäden, therapeutischer Gabe von IL-2 oder Asthma.

Als Ergebnis einer erhöhten Flüssigkeitsansammlung im Gewebe oder Organ, z.B. der Lunge, wird der notwendige Gasaustausch behindert oder völlig eingeschränkt. Es kommt kein Sauerstoff aus der Atemluft in das Blut, sodass durch Sauerstoffmangel lebensgefährliche Organschäden auftreten können.

Es gibt keine generelle Standardtherapie zur Behandlung für das Permeabilitätsödem. Allgemein bemüht man sich Patienten mit einem Lungenödem künstlich zu beatmen, um die Zufuhr von Sauerstoff ins Blut und somit zu den Organen zu gewährleisten.

Aus der DE 38 41 759 sind einzelne, vom TNF abgeleitete Peptide bekannt.

Von Carswell et al. in Proc. Natl. Acad. Sci. USA 72, 3666, 1975 wurde berichtet, dass das Serum von Endotoxin-behandelten Tieren, die zuvor mit dem Mycobacterien-Stamm Calmette-Guerin (BCG) infiziert worden waren, eine hämorrhagische Nekrose bei verschiedenen Tumoren in der Maus bewirkte. Diese Aktivität wurde dem Tumor Nekrose Faktor (TNF) zugeschrieben. TNF zeigt auch eine zytostatische oder zytotoxische Wirkung gegenüber einer Vielzahl von transformierten Zelllinien in vitro, während normale menschliche und tierische Zelllinien davon nicht betroffen werden (M. R. Ruff et al, Lymphokines, Vol. II, Academic Press Inc., New York, 1981, pp 235-275). Die biochemische Charakterisierung und das Gen für menschlichen TNF wurde bereits beschrieben (D Pennica et al, Nature 312, 724, 1984; Aggarwal, B. B. et al, J. Biol. Chem. 260, 2334-2345, 1985; Nedwin, G.E. et al, Nucl. Acids Res. 13, 6361, 1985).

Aus diesen Daten konnte die folgende Proteinstruktur für den humanen reifen Tumor Nekrose Faktor (TNF) abgeleitet werden:
(NH₂)Val Arg Ser Ser Ser Arg Thr Pro Ser Asp Lys Pro Val Ala His Val Val Ala Asn Pro Gln Ala Glu Gly Gln Leu Gln Trp Leu Asn Arg Arg Ala Asn Ala Leu Leu Ala Asn Gly Val Glu Leu Arg Asp Asn Gln Leu Val Val Pro Ser Glu Gly Leu Tyr Leu Ile Tyr Ser Gln Val Leu Phe Lys Gly Gln Gly Cys Pro Ser Thr His Val Leu Leu Thr His Thr Ile Ser Arg Ile Ala Val Ser Tyr Gln Thr Lys Val Asn Leu Leu Ser Ala Ile Lys Ser Pro Cys Gln Arg Glu Thr Pro Glu Gly Ala Glu Ala Lys Pro Trp Tyr Glu Pro Ile Tyr Leu Gly Gly Val Phe Gln Leu Glu Lys Gly Asp Arg Leu Ser Ala Glu Ile Asn Arg Pro Asp Tyr Leu Asp Phe Ala Glu Ser Gly Gln Val Tyr Phe Gly Ile Ile Ala Leu(COOH)

Weiterhin wurde das TNF-Gen von Rind, Kaninchen und Maus beschrieben (Goeddel D. V. et al., Cold Spring Harbor Symp. Quant. Biol. 51, 597, 1986).

Neben seinen zytotoxischen Eigenschaften ist TNF mit hauptbeteiligt an entzündlichen Reaktionen (J.W. Larrick et al, Pharmac. Res. Vol. 5, No. 3, 129-139, 1988). Im Tiermodell konnte die Beteiligung von TNF beim septischen Schock (Torti F. M. et al, Science 229, 867-869, 1985) und der Graft versus Host Disease (Piguet, P F et al, J. Exp. Med. 166, 1280, 1987) gezeigt werden.

Aus biochemischen Untersuchungen ist bekannt, dass das humane TNF aus verschiedenen strukturellen Elementen besteht, wie in TABELLE 1 aufgelistet:

**TABELLE 1**

| **Strukturelement** | **Position der Aminosäuren** |
|---|---|
| β-STRAND 1 | 30-32 |
| β-STRAND 2 | 45-49 |
| β-STRAND 3 | 54-71 |
| β-STRAND 4 | 76-83 |
| β-STRAND 5 | 85-87 |
| β-STRAND 6 | 91-98 |
| β-STRAND 7 | 101-103 |
| U-Tum 8 | 104-106 |
| β-STRAND 9 | 107-109 |
| β-STRAND 10 | 113-126 |
| β-STRAND 11 | 131-137 |
| α-HELIX 12 | 139-141 |
| β-STRAND 13 | 147-153 |

In Lucas R et al, Science (1994) Vol. 263. no. 5148, pp. 814 - 817 ist ein Peptid beschrieben, das von der Region Ser(99) bis Glu(116) des TNF abgeleitet wurde, und welches zur Behandlung von Ödemen vorgeschlagen wird. Dieses Peptid ist auch Gegenstand von WO 00/09149. Um dieses Peptid der WO 00/09149 jedoch nutzbar zu machen, musste artifiziell die Position Pro(100) durch die Aminosäure Cystein und die Position Cys(101) durch die Aminosäure Glycin ersetzt werden. Da das lineare Peptid Ser(99) bis Glu(116) keine erfindungsgemäße Wirkung hatte (Hribar M. et al., Eur. J. Immunol. (1999), Vol.29, 3105-3111; Braun C., J. Immunol. (2005), 175: 3402-3408; Fukuda N. et al. Am J Physiol Lung Cell Mol Physiol (2001) 280: L1258-L1265), musste zusätzlich die Position Glu(116) durch die Aminosäure Cystein ersetzt werden, um die Struktur zu erhalten und um einen Ringschluss zwischen den beiden Aminosäuren Cystein zu ermöglichen.

Ein Nachteil des, in WO00/09149 beschriebenen, Peptides besteht darin, dass dieses Peptid artifizielle, nämlich so nicht in TNF enthaltende Aminosäuresequenzen enthält. Ein solches, mit artifiziellen Strukturen versehenes, Peptid wird durch das menschliche Immunsystem als körperfremd erkannt. Eine wiederholte oder andauernde Verabreichung eines solchen Peptides in medikamentöser Form kann lebensbedrohende Immunreaktionen hervorrufen.

Es wurde nun überraschend gefunden, dass sich ein Protein, das aus Teilen der Aminosäuresequenz des reifen Tumor Nekrose Faktors (TNF) zusammengesetzt ist interessante biologische Eigenschaften aufweist, wobei ein solches Protein keine artifizielle Aminosäuresequenzen enthält.

In einem Aspekt stellt die vorliegende Erfindung ein Protein, ausgewählt aus den Aminosäuresequenzen **SEQ ID:NO:1**
(NH₂)Ala-Ile-Lys-Ser-Pro-Cys-Gln-Arg-Glu-Thr-Pro-Glu-Gly-Ala-Glu-Ala-Lys-Gly-Gly-Cys-Pro-Ser-Thr-His-Val(COOH); und **SEQ ID:NO:2**
(NH₂)Lys-Ser-Pro-Cys-Gln-Arg-Glu-Thr-Pro-Glu-Gly-Ala-Glu-Ala-Lys-Gly-Gly-Cys-Pro-Ser(COOH), worin jeweils ein Ringschluss durch Bindung zwischen zwei Cysteinresten vorliegt, zur Verfügung,

Der reife Tumor Nekrose Faktor (TNF) wie hierin verwendet, ist bevorzugt der humane reife Tumor Nekrose Faktor.

Ein Protein, das gemäß vorliegender Erfindung zur Verfügung gestellt wird, wird hierin auch als "Protein gemäß (nach) vorliegender Erfindung" bezeichnet.

Teile der Aminosäuresequenz des reifen Tumor Nekrose Faktors (TNF) werden hierin auch als "strukturellen Elementen des Tumor Nekrose Faktor (TNF)" bezeichnet.

Ein Protein gemäß vorliegender Erfindung ist N-terminal aus einem oder mehreren C-terminalen strukturellen Elementen des reifen Tumor Nekrose Faktor und C-terminal aus einem oder mehreren N-terminalen strukturellen Elementen des reifen Tumor Nekrose Faktor zusammengesetzt ist, z.B. in der Form eines Fusionsproteins.

Strukturellen Elemente des reifen Tumor Nekrose Faktor sind in TABELLE 1 definiert.

Ein Protein gemäß vorliegender Erfindung schliesst ein Fusionsprotein ein, zusammengesetzt aus Teilen der Aminosäuresequenz des humanen TNF wie oben definiert, z.B. aus strukturellen Elementen des TNF.

Es wurde weiterhin gefunden, dass es besonders vorteilhaft ist es, wenn ein Protein gemäß vorliegender Erfindung N-terminal aus den C-terminalen strukturellen Elementen β-Strand 6 bis β-Strand 10, oder β-Strand 8 bis β-Strand 9. des TNF, und C-terminal aus den N-terminalen strukturellen Elementen β-Strand 2 bis β-Strand 3, oder β-Strand 3 des TNF abgeleitet ist, mit der Maßgabe, dass zumindest zwei Cysteinreste enthalten sind.

Gemäß vorliegender Erfindung wurden besonders geeignete Proteine mit den Aminosäuresequenzen SEQ ID:NO:1 und SEQ ID:NO:2, worin jeweils ein Ringschluss durch Bindung zwischen zwei Cysteinresten vorliegt, gefunden.

In einem Protein mit der Aminosäuresequenz SEQ ID:NO 1 leitet sich der N-terminale Anteil des Proteins Ala(96) bis Lys(112) aus den C-terminalen strukturellen Elementen β-Strand 6 bis β-Strand 9 des humanen TNF ab, und der C-terminale Anteil Gly(68) bis Val(74) aus dem N-termialen strukturellen Element β-Strand 2 und β-Strand 3 des humanen TNF ab. Die Ziffern (96), (112), (68) und (74) bezeichnen die Positionen der Aminosäuren im humanen TNF.

In einem Protein mit der Aminosäuresequenz SEQ ID:NO 2 leitet sich der N-terminale Anteil des Proteins Lys(98) bis Lys(112) aus den C-terminalen strukturellen Elementen β-Strand 7 bis β-Strand 9 des humanen TNF, und der C-terminale Anteil des reversen Fusionsproteins Gly(68) bis Ser(71) aus dem N-termialen strukturellen Element β-Strand 3 des humanen TNF ab. Die Ziffern (98), (112), (68) und (71) bezeichnen die Positionen der Aminosäuren im humanen TNF.

Weiterhin wurde überraschenderweise gefunden, dass in einem Protein gemäß vorliegender Erfindung ein Ringschluss durch Bindung zwischen zwei Cysteinresten vorliegt, z.B. ein Ringschluss durch Bindung zwischen einem Cysteinrest, der aus der N-terminalen Aminosäuresequenz des TNF stammt, mit einem Cysteinrest, der aus der C- terminalen Aminosäuresequenz des TNF stammt; z.B. ein Ringschluss durch eine Disulfidbindung zwischen den jeweiligen Schwefelmolekülen der beiden Cysteinreste.

In den Proteinen SEQ ID NO:1 UND SEQ ID NO:2 kommt es zwischen den beiden Cysteinen, die den Cysteinen Cys(101) und Cys(69) im humanen TNF entsprechen, zu einem Ringschluss.

Eine Disulfidbindung kann z.B. hydrolytisch oder enzymatisch aufgespaltet werden, und ob ein Protein gemäß vorliegender Erfindung in zyklischer oder nicht-zyklischer Form vorliegt, hängt von den Umgebungsbedingungen ab.

Ein Protein gemäß vorliegender Erfindung liegt in reiner, isolierter Form in zyklischer Form vor.

In der Struktur des humanen TNF ist kein Ringschluss durch Disulfidbindung zwischen den jeweiligen Schwefelmolekülen zweier Cysteinreste ausgebildet.

Ein Protein gemäß vorliegender Erfindung kann in freier Form vorliegen, oder in der Form eines Salzes, z.B. in der Form eines Säureadditionssalzes, wie ein Acetatsalz, oder ein Trifluoressigsäuresalz und in einem weiteren Aspekt stellt die vorliegende Erfindung ein Protein gemäß vorliegender Erfindung in der Form eines Salzes zur Verfügung.

Ein Protein gemäß vorliegender Erfindung kann in geeigneter Weise hergestellt werden, z.B. analog zu einem bekannten Verfahren, wie durch chemische Synthese mittels Peptidchemie oder mittels mikrobieller Verfahren, beispielsweise wie hierin beschrieben.

Es hat sich gezeigt, dass ein Protein gemäß vorliegender Erfindung interessante biologische Aktivität zeigt und daher als Medikament verwendet werden kann.

In einem weiteren Aspekt stellt die vorliegende Erfindung ein Protein gemäß vorliegender Erfindung zur Verwendung als ein Medikament zur Verfügung, z.B. Die Verwendung eines Proteins gemäß vorliegender Erfindung als ein Medikament.

Beispielsweise zeigen biologische Untersuchungen an humanen Zellen, dass ein Protein gemäß vorliegender Erfindung, auch im Unterschied zum (humanen) TNF, praktisch keine entzündlichen oder toxischen Eigenschaften aufweisen. Zur Untersuchung werden in laborüblicher Weise humane Immunzellen aus dem Blut mit Protein gemäß vorliegender Erfindung in geringer Konzentration gemischt und inkubiert. Anschließend werden mittels üblichen Methoden Markerproteine für Entzündungen bestimmt. Trotz Zugabe eines Proteins der Aminosäuresequenz SEQ ID NO:1 oder SEQ ID NO:2, worin jeweils ein Ringschluss durch Bindung zwischen zwei Cysteinresten vorliegt, gemäß vorliegender Erfindung können solche Entzündungsproteine, wie z.B. der Entzündungsmarker Interleukin-6 (IL-6), nicht nachgewiesen werden.

Es wird erwartet, dass ein Protein gemäß vorliegender Erfindung in einem Verfahren zur Vermeidung von Entzündungen, z.B. zur Vermeidung der Bildung von Entzündungsmarkern, wie IL-6, bei der medizinischen Anwendung von Proteinen, die vom Tumor Nekrosis Faktor, z.B. vom humanen Tumor Nekrosis Faktor, abgeleitet sind, verwendet werden kann.

Es ist weiterhin eine laborübliche Methode und zum Beispiel in Clunes M.T. et al, J Physiol Volume 557, Number 3, 809-819 (June 15, 2004) beschrieben, die Aktivierung von Ionenkanälen mittels Patch-Clamp Experimenten nachzuweisen. Für Patch-Clamp Untersuchungen von Ionenkanälen wird eine Glaskanüle dünn ausgezogene und mit neutraler Pufferlösung gefüllt. Die Glaskanüle (Patch-Clamp-Pipette) wird vorsichtig auf eine intakte epitheliale Zelle gedrückt. Unterhalb der Pipette befindet sich ein Stück Membran. Zwischen dem Inneren der Pipette und der Außenlösung entsteht dadurch ein elektrischer Widerstand. In die Pipettenlösung taucht eine Elektrode, die an einen empfindlichen Verstärker angeschlossen ist.

Es ist nun überraschend zu finden, dass ein Protein der Aminosäuresequenz SEQ ID NO:1 oder SEQ ID NO:2, worin jeweils ein Ringschluss durch Bindung zwischen zwei Cysteinresten vorliegt, gemäß vorliegender Erfindung die epithelialen Ionenkanäle aktiviert, was durch die Veränderung der elektrischen Spannung vs. Stromstärke nachweisbar ist.

Zur Simulation eines Akuten Lungenschadens und zur Bildung eines Lungenödems kann in laborüblicher Weise die Lunge von Versuchstieren, z.B. Mäusen oder Ratten, mehrmals mit angesäuerter Kochsalzlösung gespült werden (zum Beispiel gemäß Isik F. et al., Eur J Cardiothorac Surg (2005); 28: 301-305). Dadurch kommt es zur Herabsetzung der Lungenfunktion. Wird nun in die Lunge der Versuchstiere ein Proteins der Aminosäuresequenz SEQ ID NO:1 oder SEQ ID NO:2, worin jeweils ein Ringschluss durch Bindung zwischen zwei Cysteinresten vorliegt, gemäß vorliegender Erfindung als Nebel oder in wässriger Lösung injiziert, so kommt es innerhalb von 3 bis 5 Stunden zu einer deutlichen Verbesserung der Lungenfunktion, angezeigt durch gestiegenen Sauerstoffgehalt im arteriellen Blut.

Somit kann ein Protein gemäß vorliegender Erfindung zur Behandlung von Ödemen, wie Lungenödemen, verwendet werden.

Es wird erwartet, dass ein Protein gemäß vorliegender Erfindung auch zur Behandlung weiterer Krankheiten, die mit der Lungenfunktion assoziiert sind, eingesetzt werden kann,. z. .B. zur Herstellung eines Medikamentes zur Behandlung von Krankheiten, die mit der Lungenfunktion assoziiert sind.

Die Behandlung von Krankheiten, die mit der Lungenfunktion assoziiert sind, schließt z.B. die Aktivierung epithelialer Ionenkanäle, die Verbesserung der Lungenfunktion und/oder die Behandlung von Ödemen, wie Lungenödemen,
die Behandlung
- der Akuter Lungen Verletzung (engl. Acute Lung Inj ury, ALI),
- des Akuter Respiratorischen Distress Syndrom (engl. Acute Respiatory Distress Syndrome, ARDS),
- des schweren akuten Atemnotsyndrom (SARS),
- der Pneumonie,
- bei Multiorganversagen,
- bei Beatmung-induzierter Lungenschäden, bei Lungentransplantationen, Transfusionassoziierterlungenschäden, therapeutischer Gabe von IL-2 oder Asthma
ein, z.B. die Aktivierung epithelialer Ionenkanäle, die Verbesserung der Lungenfunktion und/oder die Behandlung von Ödemen, wie Lungenödemen.

In einem anderen Aspekt stellt die vorliegende Erfindung ein Protein gemäß vorleigender Erfindung zur Verwendung bei der Behandlung von Lungenödemen zur Verfügung, wobei erwartet wird, dass ein Protein gemäß vorliegender Erfindung neben der Verwendung zur Behandlung von Lungenödemen auch bei der Behandlung weiteren Krankheiten, die mit der Lungenfunktion assoziiert sind, durch Verabreichung in eine ausreichende Menge an einen Patienten, der einer solchen Behandlung bedarf, verwendet werden kann.

Ein Patient, wie hierin verwendet, schließt Säugetiere, z.B. Menschen ein.

Ein Protein gemäß vorliegender Erfindung kann in der Form einer pharmazeutischen Zubereitung verabreicht werden.

In einem anderen Aspekt stellt die vorliegende Erfindung ein e pharmazeutischen Zubereitung zur Verfügung, die dadurch gekennzeichnet ist, dass sie ein Protein gemäß vorliegender Erfindung umfasst, z.B. in Verbindung mit zumindest einem pharmazeutisch akzeptablen Hilfsstoff, wie Träger oder Verdünnungsmittel, beispielsweise Füllstoffe, Bindemittel, Verbesserer der Fließeigenschaften, Gleitmittel, Geschmacksstoffe, Zucker oder Süßstoffe, Geruchsstoffe, Konservierungsstoffe, stabilisierend wirkende Stoffe, Netzmittel, Emulgatoren, Lösungsvermittler, Salze zur Regulierung des osmotischen Drucks und/oder Puffer(mischungen).

Die geeignete Menge eines Proteins gemäß vorliegender Erfindung zur Behandlung von Krankheiten wird natürlich sehr von verschiedenen Parametern abhängen, beispielsweise der chemischen Natur und der Pharmakokinetik des verwendeten Proteins, dem individiuellen Patienten, der zu behandelnden Krankheit, der Art der Anwendung, aber eine erfolgreiche tägliche Dosis in größeren Säugetieren schließt beispielsweise eine Menge von 0.0001 g bis 1.5 g ein, z.B. 0.001 mg/kg Körpergewicht bis etwa 20 mg/kg Körpergewicht, ein. Die Anwendung kann enteral oder parenteral erfolgen und erfolgt bevorzugt parenteral. Eine pharmazeutische Zubereitung gemäß vorliegender Erfindung kann in geeigneter Weise hergestellt werden, z.B. analog einer bekannten Methode, z.B. durch Misch-, Granulier-, Beschichtungs-, Lösungs-, Lyophilisierungsverfahren.

### Beschreibung der Abbildungen

Fig. 1A zeigt das HPLC Chromatogramm des Proteins mit der Aminosäuresequenz SEQ ID NO:1. Einheiten: y-Achse: Absorption in mV; x-Achse: Zeit in Minuten.
Fig. 1B zeigt das HPLC Chromatogramm des Proteins mit der Aminosäuresequenz SEQ ID NO:2. Einheiten: y-Achse: Absorption in mAU x-Achse: Zeit in Minuten.
Fig. 2A, rechte Abbildung, zeigt die Aktivierung von Natrium Ionenkanälen durch ein Protein der Aminosäuresequenz SEQ ID NO:1,

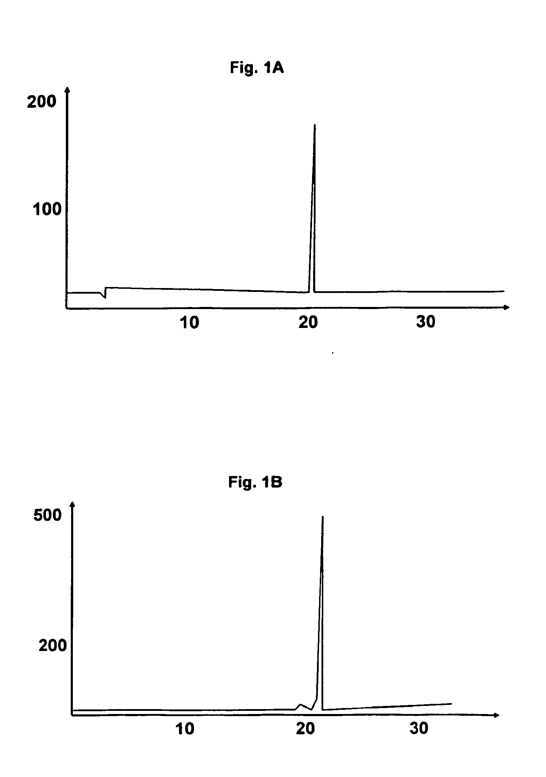

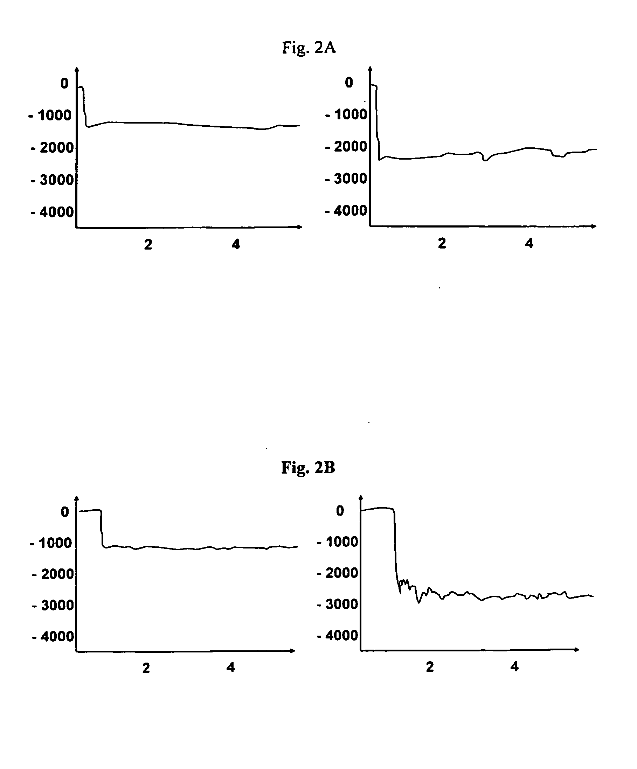

In den Fig. 1A, 1B und 2a sind die Proteine Proteine der Aminosäuresequenz SEQ ID NO: 1 oder SEQ ID NO:2, worin jeweils ein Ringschluss durch Bindung zwischen zwei Cysteinresten vorliegt, detektiert durch Patch Clamp. Linke Abbildung in der Fig. 2A im Vergleich. ohne Protein. Einheiten: y-Achse: Stromstärke in pA; x-Achse: Zeit in Sekunden.

Fig. 2B, rechte Abbildung, zeigt die Aktivierung von Natrium Ionenkanälen durch ein Protein der Aminosäuresequenz SEQ ID NO:2, detektiert durch Patch Clamp. Linke Abbildung in der Fig. 2B im Vergleich ohne Protein. Einheiten: y-Achse: Stromstärke in pA; x-Achse: Zeit in Sekunden.

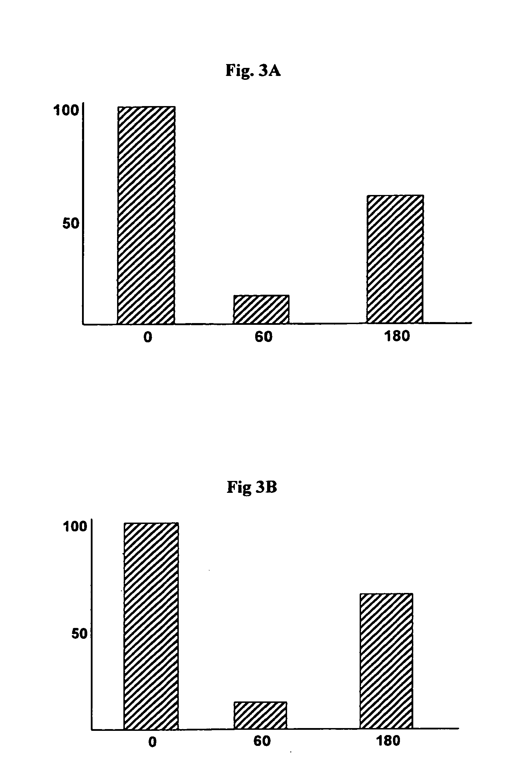

Fig. 3A zeigt die Erhöhung des Sauerstoffgehalts im arteriellen Blut nach Verabreichung eines Proteins mit der Aminosäuresequenz SEQ ID NO:1. Einheiten: y-Achse: Sauerstoffegehalt in %; x-Achse: Messzeitpunkt in Minuten.

Fig. 3B zeigt die Erhöhung des Sauerstoffgehalts im arteriellen Blut nach Verabreichung eines Proteins mit der Aminosäuresequenz SEQ ID NO:2. Einheiten: y-Achse: Sauerstoffegehalt in %; x-Achse: Messzeitpunkt in Minuten.

In den Beispielen werden folgende Abkürzungen verwendet:

| | |
|---|---|
| TFA Salz | Salz der Trifluoressigsäure |

Die isolierten Proteine der Aminosäuresequenz SEQ ID NO:1 oder SEQ ID NO:2 sind zuklische Proteine, worin jeweils ein Ringschluss durch Bindung zwischen zwei Cysteinresten vorliegt,

### Beispiel 1: Synthese eines Proteins mit der mit der Aminosäuresequenz SEQ ID NO:1

Ein Protein mit der Aminosäuresequenz SEQ ID NO:1 wurde mittels Fmoc-Festphasensynthese vollautomatisch in folgenden Schritten synthetisiert:

| **Schritt** | **Prozess** | **Produkt** |
|---|---|---|
| 1 | Kopplung der Aminosäuren | Peptid gebunden an die Festphase |
| 2 | Abspaltung von der Festphase | Peptid in Lösung |
| 3 | Reinigung | Gereinigtes Peptid als TFA-Salz |
| 4 | Reinigung / Salz Austausch / oxidative Zyklisierung | Gereinigtes Peptid als Acetatsalz |
| 5 | Analytische Untersuchung | Gereinigtes Peptid |

Die Zyklisierung wurde durch Herausbildung einer Disulfidbrücke zwischen den Seitenketten der Aminosäuren Cystein (Position 6) und Cysten (Position 20) erreicht. Dies erfolgt zum Beispiel durch Sauerstoffoxidation der Schwefelatome in den Seitenketten des Cysteins (Position 6) und des Cystens (Position 20) unter Herausbildung einer Disulfidbrücke, was zum Ringschluss führt.

Anschließend wurde das Protein mittels Reverser HPLC untersucht, wobei das Ergebnis, wie in Fig. 1A gezeigt, erhalten wurde.

### Beispiel 2: Synthese eines Proteins mit der Aminosäuresequenz SEQ ID NO:2

Ein Protein mit der Aminosäuresequenz SEQ ID NO:2 wurde mittels Fmoc-Festphasensynthese vollautomatisch in folgernden Schritten synthetisiert:

| **Schritt** | **Prozess** | **Produkt** |
|---|---|---|
| 1 | Kopplung der Aminosäuren | Peptid gebunden an die Festphase |
| 2 | Abspaltung von der Festphase | Peptid in Lösung |
| 3 | Reinigung | Gereinigtes Peptid als TFA-Salz |
| 4 | Reinigung / Salz Austausch / oxidative Zyklisierung | Gereinigtes Peptid als Acetatsalz |
| 5 | Analytische Untersuchung | Gereinigtes Peptid |

Die Zyklisierung wurde durch Herausbildung einer Disulfidbrücke zwischen den Seitenketten der Aminosäuren Cystein (Position 4) und Cysten (Position 18) erreicht. Dies erfolgt zum Beispiel durch Sauerstoffoxidation der Schwefelatome in den Seitenketten des Cysteins (Position 4) und des Cystens (Position 18) unter Herausbildung einer Disulfidbrücke, was zum Ringschluss führt.

Anschließend wurde das Protein mittels Reverser HPLC untersucht, wobei das Ergebnis, wie in Fig. 1B gezeigt, erhalten wurde:

### Beispiel 4: Zellkultur

Die elektrophysiologischen Experimente wurden an humanen A549 Zellen (ATTC Nr. CCL-185) durchgeführt. A549 Zellen sind humane Lungenepithelzellen, die an der Diffusion von Wasser und Elektrolyten in der Lunge beteiligt sind.

Die Zellen wurden in DMEM-F-12 Medium mit 1% Penicillin-Streptomycin und 10% fötalem Kälberserum suspendiert, in Plastizellkulturgefäße überführt und im Inkubator bei 95% Luft und 5% CO₂ bei 37°C kultiviert. Das Medium wurde 2- bis 3-mal in der Woche gewechselt. Die Zellen verdoppeln sich in ca. 22 Stunden und eine Zellkonzentration von über 7 x 10⁴ Zellen pro cm² wurde nicht überschritten.

### Beispiel 5: Aktivierung von Ionenkanälen humaner Epithelzellen durch Proteine mit den Aminosäuresequenzen SEQ ID NO:1 oder SEQ ID NO:2

Makroskopische Ströme und Einzelkanalströme wurden von A549 Zellen in der "whole cell" und "cell-attached" Konfiguration der"Patch-clamp" Technik (Hamill et al, Pflugers Arch. 1981, 391(2):85-100., 1981) abgeleitet. Für die Stromableitungen in der "whole cell" Konfiguration wurden folgende Bad- und Elektrodenlösungen verwendet:
Badlösung: 135 mM Natrium Methansulfonat, 10 mM NaCl, 2.7 mM KCl, 1.8 mM CaCl2, 2 mM MgCl2, 5.5 mM Glucose, und 10 mM HEPES, pH 7.4.
Elektrodenlösung: 120 mM Kaliummethylsulfonat, 15 mM KCl, 6 mM NaCl, 1 mM Mg2ATP, 2 mM Na3ATP, 10 mM HEPES, and 0.5 mM EGTA (pH 7.2).

Die Deckgläser mit den darauf kultivierten Zellen wurden in ein 1 ml fassendes Versuchsbad transferiert, auf dem Mikroskoptisch (Axiovert 100, 400-fache Vergrößerung) fixiert und die Zellen mit der oben beschriebenen Badlösung superfundiert. Sodann wurde von einer geeigneten Zelle (welche am Deckglas haftet) der Strom abgeleitet. Dazu wurde eine mit einer Elektrolytlösung gefüllte Mikroelektrode (Glaskapillare mit einer definierten, Hitzepolierten Spitzenöffnung von ca. 1-3 µm, entspricht einen Widerstand der Elektrodenspitze von 3-5 Ω) auf die Zelle aufgesetzt und die Membran angesaugt, sodass ein "Gigaohm-Seal" zwischen Membran und Elektrode gebildet wurde, um den Leckstrom zu minimieren. In der "cell-attached"-Konfiguration kann der Strom durch einzelne Ionenkanäle unter der Elektrodenspitze gemessen werden. Bei der "whole cell"-Konfiguration wurde die Membran unter der Elektrodenspitze durchbrochen, damit der Strom, der durch alle Ionenkanäle der Zelle fließt, gemessen werden kann. Die Ableitung der makroskopischen Ströme kann auch mit Hilfe der "perforated patch clamp" Technik durchgeführt werden. Bei der "whole cell" Ableitung wurde der Ionophor Amphotericin der Pipettenlösung zugesetzt, wodurch die Membran unter der Spitzenöffnung durchlässig wurde und Ströme in der "whole cell" Konfiguration abgeleitet werden können. Bei Erhalt eines Gigaohm-Seals wurde über einen Vorverstärker (CV-4 Headstage, Axon Instruments) und Verstärker (Axopatch 1D, Axon Instr.) ein definiertes Membranhaltepotential angelegt und der Strom, der dabei durch die Ionenkanäle fließt, gemessen.

Das Pulsprotokoll bestand aus einer Hyperpolarisation auf -100 mV für 1 s im Abstand von 5 s. In weiterer Folge wurde dann in Schritten von 20 mV schließlich die Membran bis zu +100 mV depolarisiert. Dieses Protokoll wurde unter Zugabe von synthetischen Proteinen mit den Aminosäuresequenzen SEQ ID NO:1 oder SEQ ID NO:2, sowie mit dem Natriumkanal-Inhibitor Amilorid durchgeführt. Die so erhaltenen Stromableitungen wurden gespeichert und mit Hilfe des Programms PCLAMP 6.0 analysiert. Dazu wurden die, unter Anwesenheit von Amilorid erhaltenen, Stromableitungen von den vorher registrierten Strömen subtrahiert, sodass der Amilorid-sensitive Natriumstrom durch die epithelialen Natriumkanäle ermittelt werden konnte.

Die Resultate, in denen die Aktivierung der Natrium Ionenkanäle durch die Proteine mit den Aminosäuresequenzen SEQ ID NO:1 und SEQ ID NO:2 gezeigt wird, sind aus Fig. 2A und Fig. 2B ersichtlich.

### Beispiel 6: Experimentelle Tierstudie Lungenödem

Männliche Wistar Ratten (Gewicht 250 g bis 350 g) werden mit Rompun® (0,02 ml/100g) und Ketavet® (0,1 ml/100g) anästhesiert. Die Beatmung erfolgt mit einem Zyklus von 72 Stößen / Minute, bei einer Einatmungszeit von 0,2 Sekunden und einer Ausatmungszeit von 0,5 Sekunden. Die Körpertemperatur beträgt durchschnittlich 37°C bis 39°C. Im Normalzustand beträgt der PaO2 (arterieller Sauerstoffpartialdruck) 500 bis 550 mm Hg. Zur Simulation eines Akuten Lungenschadens und zur Bildung eines Lungenödems wird die Lunge 7 bis 9 mal mit angesäuerter Kochsalzlösung (pH 5) gespült.

Nach einer Stunde werden jeweils die Proteine mit der Aminosäuresequenz SEQ ID NO:1 oder SEQ ID NO:2, gelöst in steriler Kochsalzlösung, intratracheal als Nebel verabreicht (Maximales verabreichtes Volumen 0,5 ml).

In einem Abstand von jeweils 60 Minuten wird den Tieren arterielles Blut entnommen (0,1 ml) und der Sauerstoffgehalt in % zum Normalwert bestimmt.

Nach Verabreichung eines Proteins mit der Aminosäuresequenz SEQ ID NO:1 oder SEQ ID NO:2 ist der Sauerstoffanteil im Blut erhöht, wie aus Fig. 3A oder Fig.3B ersichtlich, siehe auch Beispiel 7.

### Beispiel 7: Verbesserung der Lungenfunktion

Der Nachweis der stimulierenden Wirkung eines Proteins mit der Aminosäuresequenz SEQ ID NO:1 oder SEQ ID NO:2 gemäß vorliegender Erfindung auf die Lungenfunktion erfolgt mittels tierexperimentellen Studien, in welchem ein Lungenödem induziert wird. Die experimentelle Vorgehensweise ist im Beispiel 6 beschrieben. Zur Objektivierung der Messwerte werden jeweils 5 Tiere verwendet.

Zur intratrachealen Inhalation werden jeweils 125 µg Protein in 150 mM Kochsalzlösung pH 7,3 aufgelöst. Der Sauerstoffgehalt des arteriellen Blutes wird unmittelbar vor der Spülung der Lungen, 60 Minuten nach der Spülung der Lungen und 180 Minuten nach Spülen der Lunge gemessen.

Der Sauerstoffgehalt unmittelbar vor dem Lungenspülen wird mit 100% festgelegt. 60 Minuten nach dem jeweils letzen Lungenspülen beträgt der Sauerstoffgehalt im Blut durchschnittlich nur 20%. Innerhalb von 3 Stunden erhöhte sich der prozentuale Sauerstoffgehalt auf Werte von

60% bei Behandlung mit einem Protein mit der Aminosäuresequenz SEQ ID NO:1, bzw. 63% bei Behandlung mit einem Protein mit der Aminosäuresequenz SEQ ID NO:2

Ohne Zugabe von Protein kommt es innerhalb von 180 Minuten nach Lungenspülung zu keiner Verbesserung der Lungenfunktion (Sauerstoffgehalt 20%).

Die Resultate sind dargestellt in
- Fig. 3A für ein Protein mit der Aminosäuresequenz SEQ ID NO:1,
- Fig. 3B für ein Protein mit der Aminosäuresequenz SEQ ID NO:2,

## Patentansprüche

1. Protein, ausgewählt aus den Aminosäuresequenzen
SEQ ID:NO:1 (NH₂)Ala-Ile-Lys-Ser-Pro-Cys-Gln-Arg-Glu-Thr-Pro-Glu-Gly-Ala-Glu-Ala-Lys-Gly-Gly-Cys-Pro-Ser-Thr-His-Val(COOH); und
SEQ ID:NO:2 (NH₂)Lys-Ser-Pro-Cys-Gln-Arg-Glu-Thr-Pro-Glu-Gly-Ala-Glu-Ala-Lys-Gly-Gly-Cys-Pro-Ser(COOH),
worin jeweils ein Ringschluss durch Bindung zwischen zwei Cysteinresten vorliegt.

2. Protein nach Anspruch 1 in der Form eines Salzes.

3. Protein gemäß einem der Ansprüche 1 oder 2, zur Verwendung als Medikament.

4. Protein gemäß einem der Ansprüche 1 bis 3, zur Verwendung bei der Behandlung von Lungenödemen.

5. Pharmazeutischen Zubereitung, **dadurch gekennzeichnet, dass** sie ein Protein gemäß einem der Ansprüche 1 oder 2 umfasst.

## Claims

1. A band, in particular a wrist band, having a clasp (10) for varying the length (11) of the band formed by two band portions (1, 2), wherein the clasp (10) may be adjusted at a first band portion (1) and may be releasably attached at a second band portion (2) and wherein the first band portion (1) partially overlaps the second band portion (2) in the position of the clasp (10) being attached at the second band portion (2), **characterized in that** the clasp (10) is hinged at a free end of a connection section (6) adjoining the second band portion (2), wherein the connection section (6) that is attached to the clasp (10) and adjoining to the second band portion (2) may be rolled from an extended position into a partially overlapping position, wherein in a reduced position of the length (11) of the band, the first band portion (1) partially covers the second band portion (2) at the external surface (8) thereof and completely covers the rollable connection section (6) as well as the second band portion (2) at least partially covers the internal surface of the connection section (6).

2. A band having a clasp (10) according to claim 1, **characterized in that** in the reduced position of the length (11) of the band, at least one portion of the connection section (6) is directly supported on the external surface (8) of the second band portion (2).

3. A band having a clasp (10) according to claim 1 or 2, **characterized in that** the first band portion (1) has a series of holes (4) that extend centrally in its width and that the clasp (10) may be attached at the first band portion (1) by way of an attachment peg (25) that is provided at one clasp basic body (22), which passes through a hole (5) of the series of holes (4).

4. A band having a clasp (10) according to any of claims 1 to 3, **characterized in that** there is provided a peg (12) at the external surface (8) of the second band portion (2), the axis of which extending vertically from the external surface (8) of the second band portion (2), and which preferably has a circumferential bulge (13) at the free end thereof.

5. A band having a clasp (10) according to claim 4, **characterized in that** the peg (12) is attached at a plate (14), wherein the plate (14) is preferably covered by a cover layer (16) of the second band portion (2).

6. A band having a clasp (10) according to claim 4, **characterized in that** the peg (12) may be attached in a hole (5) of a series of holes (4), extending centrally alongside the width thereof, of the second band portion (2), for example being screwed or plugged in.

7. A band having a clasp (10) according to any of claims 1 to 6, **characterized in that** in the reduced position of the length (11) of the band, the peg (12) that vertically projects from the external surface (8) of the second band portion (2) may be accommodated by a bore (34) at the bottom side (24) of the clasp basic body (22) and may be fixed by way of the clasp (10).

8. A band having a clasp (10) according to any of claims 1 to 7, **characterized in that** the two band portions (1, 2) are made in one or several layers, e.g., from leather, plastic, elastomer, textile fabrics or combinations thereof and that a connection section (6) optionally adjoining the second band portion (2) is preferably made from a soft and flexible material, in particular elastomer.

9. A clasp (10) for varying the length (11) of a band formed by two band portions (1, 2), in particular a wrist watch band, wherein the clasp (10) comprises a movable clasp flap (17) and a clasp basic body (22), wherein a first band portion (1) may be adjustably attached by means of a series of holes (4) extending centrally alongside the width thereof at an attachment peg (25) provided at the top surface (23) of the clasp basic body (22) of the clasp (10), which passes through a hole (5) of the series of holes (4) of the band portion (1), wherein the clasp flap (17) serves for fixing the adjustable attachment of the first band portion (1) with the clasp (10), **characterized in that** a second band portion (2) may be attached by means of peg (12) provided at the external surface (8) thereof and projecting vertically therefrom at the clasp basic body (22), wherein the peg (12) may be fixed in a bore (34) at the bottom surface (24) of the clasp basic body (22) of the clasp (10).

10. A clasp (10) according to claim 9, **characterized in that** the peg (12) may be magnetically fixed in a bore (34) at the bottom surface (24) of the clasp basic body (22) of the clasp (10).

11. A clasp (10) according to claim 9 or 10, **characterized in that** for fixing the peg (12) of the second band portion (2) in the bore (34) of the clasp basic body (22) in a free cross-section (30) within the clasp basic body (22), there are movably arranged two lever locks (38), the locking hook (39) thereof preventing unintentional opening of the peg (12) preferably provided with a bulge (13) by way of locking springs (43).

12. A clasp (10) according to any of claims 9 to 11, **characterized in that** the clasp basic body (22) as well as the clasp flap (17) are made preferably from metal, for example stainless steel, or plastic material, for example glass fibre reinforced plastic.

## Revendications

1. Bande, en particulier bande de bracelet-montre, comprenant un fermoir (10) pour faire varier la longueur (11) de la bande formée par deux parties de bande (1, 2), le fermoir (10) étant susceptible d'être fixé de manière réglable sur une première partie de bande (1) et de manière détachable sur une seconde partie de bande (2), et dans une situation du fermoir (10) fixé sur la seconde partie de bande (2) la première partie de bande (1) recouvre partiellement la seconde partie de bande (2), **caractérisée en ce que** le fermoir (10) est fixé de manière articulée à une extrémité libre d'un tronçon de liaison (6) adjacent à la seconde partie de bande (2), dans laquelle le tronçon de liaison (6) fixé au fermoir (10) et adjacent à la seconde partie de bande (2) est déplaçable de manière déroulante depuis une position étirée jusque dans une position en chevauchement partiel, et dans une situation réduite de la longueur (11) de la bande, la première partie de bande (1) recouvre partiellement la seconde partie de bande (2) au niveau de sa face extérieure (8) et recouvre totalement le tronçon de liaison (6) déplaçable de manière déroulante, et la seconde partie de bande (2) recouvre au moins partiellement le tronçon de liaison (6) du côté intérieur.

2. Bande avec fermoir (10) selon la revendication 1, **caractérisée en ce que** dans la situation réduite de la longueur (11) de la bande, au moins une partie du tronçon de liaison (6) est appliquée directement contre la face extérieure (2) de la seconde partie de bande (2).

3. Bande avec fermoir (10) selon la revendication 1 ou 2, **caractérisée en ce que** la première partie de bande (1) comporte une rangée de trous (4) s'étendant en longueur au milieu de sa largeur, et le fermoir (10) est susceptible d'être fixé sur la première partie de bande (1) au moyen d'un tenon de fixation (25), prévu sur le corps de base (22) du fermoir, le tenon traversant un trou (5) de la rangée de trous (4).

4. Bande avec fermoir (10) selon l'une des revendications 1 à 3, **caractérisée en ce que** sur la face extérieure (8) de la seconde partie de bande (12) il est prévu un tenon (12), dont l'axe dépasse perpendiculairement de la face extérieure (8) de la seconde partie de bande (2), et qui comprend de préférence à son extrémité libre un bourrelet périphérique (13).

5. Bande avec fermoir (10) selon la revendication 4, **caractérisée en ce que** le tenon (12) est fixé sur une plaque (14), ladite plaque (14) étant de préférence recouverte par une couche de couverture (16) de la seconde partie de bande (2).

6. Bande avec fermoir (10) selon la revendication 4, **caractérisée en ce que** le tenon (12) est susceptible d'être fixé dans un trou (5) d'une rangée de trous (4) s'étendant en longueur au milieu de sa largeur, de la seconde partie de bande (2), par exemple par vissage ou fixé de manière enfichable.

7. Bande avec fermoir (10) selon l'une des revendications 1 à 6, **caractérisée en ce que** dans la situation réduite de la longueur (11) de la bande, le tenon (12) qui dépasse perpendiculairement de la face extérieure (8) de la seconde partie de bande (2) est susceptible d'être reçu par un perçage (34) à la face inférieure (24) du corps de base (22) du fermoir et susceptible d'être fixé avec le fermoir (10).

8. Bande avec fermoir (10) selon l'une des revendications 1 à 7, **caractérisée en ce que** les deux parties de bande (1, 2) sont fabriquées à une ou à plusieurs couches, par exemple en cuir, en matière plastique, en élastomère, en tissu textile ou leurs combinaisons, et un tronçon de liaison (6) éventuellement adjacent à la seconde partie de bande (2) est fabriqué de préférence en un matériau flexible souple, en particulier en élastomère.

9. Fermoir (10) pour faire varier la longueur (11) d'une bande formée par deux parties de bande (1, 2), en particulier d'une bande de bracelet-montre, dans lequel le fermoir (10) comprend un clapet de fermoir (17) mobile et un corps de base de fermoir (22), dans lequel une première partie de bande (1) est susceptible d'être fixée de manière réglable au moyen d'une rangée de trous (4), s'étendant en longueur au milieu de sa largeur, sur un tenon de fixation (25), prévu sur la face supérieure (23) du corps de base (22) du fermoir (10), qui traverse un trou (5) de la rangée de trous (4) de la partie de bande (1), dans lequel le clapet de fermoir (17) sert à la fixation réglable de la première partie de bande (1) avec le fermoir (10), **caractérisé en ce qu'**une seconde partie de bande (2) est susceptible d'être fixée sur le corps de base (22) du fermoir au moyen d'un tenon (12) prévu sur sa face extérieure (8) et dépassant perpendiculairement de cette face, dans lequel le tenon (12) est susceptible d'être fixé dans un perçage (34) à la face inférieure (24) du corps de base (22) du fermoir (10).

10. Fermoir (10) selon la revendication 9, **caractérisé en ce que** le tenon (12) est susceptible d'être fixé de manière magnétique dans un perçage (34) à la face inférieure (24) du corps de base (22) du fermoir (10).

11. Fermoir (10) selon la revendication 9 ou 10, **caractérisé en ce que** pour la fixation du tenon (12) de la seconde partie de bande (2) dans le perçage (34) du corps de base (22) du fermoir, deux leviers de fermeture (38) sont agencés de manière mobile dans une section libre (30) à l'intérieur du corps de base (22) du fermoir, leviers dont les crochets de verrouillage (39) assurent une protection vis-à-vis d'une ouverture inopinée du tenon (12) doté de préférence d'un bourrelet (13), au moyen de ressorts de fermeture (43).

12. Fermoir (10) selon l'une des revendications 9 à 11, **caractérisé en ce que** le corps de base (22) du fermoir ainsi que le clapet de fermoir (17) sont fabriqués de préférence en métal, par exemple en acier inoxydable, ou bien en matière plastique, par exemple en matière plastique renforcée par des fibres de verre.
